# EUROPEAN PATENT SPECIFICATION

(11) **EP 2 340 767 B1**
(45) Date of publication and mention of the grant of the patent: **23.08.2017**
(21) Application number: 11002306.6
(22) Date of filing: 29.12.2005
(51) Int. Cl.: A61B 1/00, A61B 1/005, A61B 8/00, A61B 8/12

(54) **ULTRASOUND ENDOSCOPE**
ULTRASCHALL ENDOSKOP
ENDOSCOPE A ULTRASONS

(30) Priority: 07.02.2005 JP 2005031132; 03.08.2005 JP 2005225571
(43) Date of publication of application: 06.07.2011
(62) Divisional of application: 05822706.7
(73) Proprietor: Olympus Corporation, Hachioji-shi Tokyo 192-8507 (JP)
(72) Inventor: Imahashi, Takuya, Tokyo 151-0072 (JP); Wakabayashi, Katsuhiro, Tokyo 151-0072 (JP); Sawada, Yukihiko, Tokyo 151-0072 (JP); Mizunuma, Akiko, Tokyo 151-0072 (JP); Aoki, Hidemichi, Tokyo 151-0072 (JP); Sato, Sunao, Tokyo 151-0072 (JP)
(74) Representative: Gunzelmann, Rainer

(56) References cited:
- JP-A- 9 135 833
- US-A- 5 476 497
- US-A1- 2002 049 485
- US-A1- 2002 062 084

## Description

### Technical Field

The present invention relates to an electronic radial ultrasound endoscope for which the operational performance is enhanced and which reduces the burden on patients and physicians.

### Background Art

In recent years, an ultrasound endoscope of the electronic scanning type, which has an ultrasonic probe at the tip of an insertion tube of the endoscope and scans electronically, has been put into practical use. In the electronic scanning type ultrasound endoscope, many piezoelectric transducers are arranged in the form of an array. These piezoelectric transducers are suitably driven with a Diagnostic ultrasound system to which the ultrasound endoscope is connected, and thereby an ultrasonic image is obtained.

For a wire for transmitting an electric signal to the ultrasonic probe, a plurality of flexible boards (FPCs : Flexible Printed Circuits) are used (for example, see Patent Documents 1 and 2).

The FPCs are arranged within a bending tube so that they can avoid contacting parts of the endoscope used for observation such as a forceps channel. Additionally, the FPCs are connected to signal lines (coaxial cables) at the tip of a flexible tube on the side nearer the bending tube (the side nearer the control section of the main body of the ultrasound endoscope) (for example, see Fig. 1 of Patent Document 2).

With the angulation control of a typical endoscope, the angulation angle in the UP direction must be great, namely, must be able to be bent at approximately 130 degrees; the angulation angle must also be at least 90 degrees in other directions. With such a configuration, insertion into a patient is made smooth, and the burden on patients and the physicians that operate the endoscope are reduced.

Alternately, if the FPCs are not comprised and signal lines are directly arranged, a number of signal lines can be bound with a heat-shrinkage tube or other such device over the total length of the rigid part of the tip, the bending tube, and the flexible tube.

In the meantime, improvements in the operational performance of ultrasound endoscopes have led to reductions in the burden on patients and physicians. Therefore, it is important that the bending tube of the tip be easy to bend in a predetermined direction. The degree of bending is significantly influenced by the cable for transmitting/receiving ultrasounds, and in particular by the shape, material, and arrangement of the cable.

In addition, shortening and making thinner the length of the rigid part inserted into a body cavity improves the operational performance, and this leads to reductions in the burden on patients and physicians.

A technique is disclosed with which the degree of freedom of a channel for inserting an endo-therapy accessorie is improved by branching or transforming an ultrasound cable in the rigid part of a tip (the distal rigid section), in which an endoscopic observation part and an ultrasound observation part are fixed, in a convex ultrasound endoscope (for example, Patent Document 3).

Additionally, a convex ultrasound endoscope having a cable inclined from a transducer is disclosed (for example, Patent Document 4).

The techniques of Patent Documents 3 and 4 do not refer to the positions of the transducers of the endoscopic observation part and the ultrasound observation part. This is because the ultrasound observation cannot be made 360 degrees even with the transducers, and both of their ends always exist in an observation range. Accordingly, the positions of the transducer elements are determined naturally.
Patent Document 1: Japanese Unexamined Published Patent Application No. 2002-153465
Patent Document 2: Japanese Unexamined Published Patent Application No. 2002-153470
Patent Document 3: Japanese Unexamined Published Patent Application No. 2001-170054
Patent Document 4: Japanese Unexamined Published Patent Application No. 2001-112757
US2002/062084 discloses an ultrasonic endoscope having a bending portion, an ultrasonic probe, and a flexible circuit board. The flexible circuit board is constructed of a plurality of flexible circuit board strips in the bending portion so as to allow a bending motion, and a plurality of ultrasonic wave coaxial lines, provided in the flexible tube, are loose and flexible in a given range.

US 5,476,497 discloses an oval electrode lead array comprising two conductors arranged inside a tube so as to have an oval configuration, wherein the lead array tends to bend about the major axis of the oval cross section.

JP 09/135833 discloses an ultrasonic endoscope having a signal cable that is inclined obliquely rearward, approaching an outside of the distal tip in a proximal direction, so as not to interfere with a downward wire retainer.

### Disclosure of Invention

An ultrasound endoscope not forming part of the present invention comprises an ultrasonic probe in which a plurality of ultrasonic transducer elements for transmitting/receiving ultrasounds are arranged in a nearly cylindrical form, a rigid part that constitutes the tip of an endoscopic insertion tube and in which the ultrasonic probe is provided, a bending tube to which the distal rigid section is connected and that bends with remote operation, a flexible tube connected to the bending tube, and a signal line bundle that is a bundle of signal lines corresponding to respective ultrasonic transducer elements for transmitting a drive signal driving each of the ultrasonic transducer elements and that passes through the insides of the rigid tip part, the bending tube, and the flexible tube. The signal line bundle is covered with a binding member for binding the signal line bundle, and the binding force of the binding member that covers the signal line bundle in a certain predetermined range in the signal line bundle included in the bending tube is weaker than the binding force of the binding member that covers the signal line bundle in the areas other than those within the predetermined range.

Additionally, an ultrasound endoscope not forming part of the present invention comprises an ultrasonic probe where a plurality of ultrasonic transducer elements for transmitting/receiving ultrasounds are arranged in a nearly cylindrical form, a rigid part that constitutes the tip of an endoscopic insertion tube and in which the ultrasonic probe is provided, a bending tube to which the distal rigid section is connected and which bends with remote operation, a flexible tube connected to the bending tube, and a signal line bundle that is a bundle of signal lines corresponding to respective ultrasonic transducer elements for transmitting a drive signal driving each of the ultrasonic transducer elements, and passes through the insides of the distal rigid section, the bending tube, and the flexible tube. The interval between signal lines of the signal line bundle in a certain predetermined range in the signal line bundle included in the bending tube is made stronger than the interval between signal lines of the signal line bundle that exist in the area other than the area within the predetermined range.

Furthermore, an electronic radial ultrasound endoscope according to claim 1 is provided. Preferable embodiments are set forth in the dependent claims. The electronic radial ultrasound endoscope may comprise an endoscopic observation part where an illumination optical system and an observation optical system are provided, an ultrasonic observation part where a plurality of ultrasonic transducer elements for transmitting/receiving ultrasounds are arranged in a rigid part that constitutes the tip of an insertion tube, and a bending tube that bends freely at least in mutually orthogonal first and second bending directions at the rear end of the distal rigid section. The thickness of a cable composed of a bundle of signal lines connected to the respective ultrasonic transducer elements is fixed to be thinner in the first bending direction than in the second bending direction in the distal rigid section.

Still further, an electronic radial ultrasound endoscope may comprise an endoscopic observation part where an illumination optical system and an observation optical system are provided, an ultrasonic observation part where a plurality of ultrasonic transducer elements for transmitting/receiving ultrasounds are arranged in a rigid part that constitutes the tip of an insertion tube, and a bending tube that bends freely at least in mutually orthogonal first and second bending directions at the rear end of the distal rigid section. A cable composed of a bundle of signal lines connected to the respective ultrasonic transducer elements is branched, and the thickness of the bundle of the branched cables is fixed to be thinner in the first bending direction than in the second bending direction in the rigid tip part.

### Brief Description of Drawings

Fig. 1 is a schematic of a signal line bundle 201 of a bending tube of a conventional endoscope;
Fig. 2 is a conceptual schematic of bound portions and a weakly bound portion of signal lines in a first preferred embodiment;
Fig. 3 is a schematic of the configuration of the external portion of an ultrasound endoscope of the inventions;
Fig. 4A is an enlarged view (external perspective view) of the tip part of the ultrasound endoscope 1 of Fig. 3;
Fig. 4B is an enlarged view of the outside of the tip part of the ultrasound endoscope 1 of Fig. 3;
Fig. 5 is a cross sectional view of an ultrasonic probe in the 1-1 preferred embodiment;
Fig. 6 is a perspective view of the ultrasonic probe in the 1-1 preferred embodiment;
Fig. 7 shows a cross section of an area of the ultrasound endoscope in the 1-1 preferred embodiment in the vicinity of an insertion tube 2;
Fig. 8 shows a cross section of a multi-core coaxial cable in the 1-1 preferred embodiment;
Fig. 9 is a schematic of the tip of an insertion tube of an ultrasound endoscope in a 1-2 preferred embodiment;
Fig. 10 shows the state of a signal line bundle when a bending tube 8 of the ultrasound endoscope in the 1-2 preferred embodiment is bent;
Fig. 11 is a schematic of the tip of an insertion tube of an ultrasound endoscope in a 1-3 preferred embodiment;
Fig. 12A shows a cable belonging to a distal rigid section of an electronic radial ultrasound endoscope in the invention;
Fig. 12B is a schematic (F-F cross sectional view) of the cable of the distal rigid section of the electronic radial ultrasound endoscope in the second preferred embodiment (implementation example 1);
Fig. 12C is a schematic (G-G cross sectional view) of the cable of the distal rigid section of the electronic radial ultrasound endoscope in the second preferred embodiment (implementation example 1);
Fig. 13A is a schematic of the cable of the distal rigid section of the electronic radial ultrasound endoscope in the second preferred embodiment (implementation example 2);
Fig. 13B is a schematic (F-F cross sectional view) of the cable of the distal rigid section of the electronic radial ultrasound endoscope in the second preferred embodiment (implementation example 2);
Fig. 13C is a schematic (G-G cross sectional view) of the cable of the distal rigid section of the electronic radial ultrasound endoscope in the second preferred embodiment (implementation example 2);
Fig. 14A is a schematic showing the cable of the distal rigid section of the electronic radial ultrasound endoscope in the second preferred embodiment (implementation example 3);
Fig. 14B is a schematic (F-F cross sectional view) showing the cable of the distal rigid section of the electronic radial ultrasound endoscope in the second preferred embodiment (implementation example 3);
Fig. 14C is a schematic (G-G cross sectional view) of the cable of the distal rigid section of the electronic radial ultrasound endoscope in the second preferred embodiment (implementation example 3);
Fig. 15 is a schematic of the cable in the second preferred embodiment with a heat-shrinkage tube attached;
Fig. 16A is a schematic of a cable fixing member 63 for fixing the cable shown in Fig. 15 (a state where Fig. 15 is viewed in the left direction);
Fig. 16B is a schematic of the cable fixing member 63 for fixing the cable shown in Fig. 15 (a state where Fig. 15 is viewed in the same direction as that of Fig. 15) ;
Fig. 16C is a schematic of the cable fixing member 63 for fixing the cable shown in Fig. 15 (a state where Fig. 15 is viewed in the right direction);
Fig. 17A is a schematic of the distal rigid section of the electronic radial ultrasound endoscope shown in Fig. 13;
Fig. 17B is an H-H cross sectional view of the distal rigid section in Fig. 17A;
Fig. 18 is a cross sectional view of the inclination of the cable of the distal rigid section in the second preferred embodiment;
Fig. 19A is a schematic of a cable fixing member (right and left directions) for inclining the cable in the second preferred embodiment; and
Fig. 19B is a schematic of a cable fixing member (up and down directions) for inclining the cable in the second preferred embodiment.

### Best Mode of Carrying Out the Invention

### <first preferred embodiment>

If a bending tube is bent significantly as described above when an FPC is used as in the prior art examples, an expansion/contraction force is exerted in the direction of the insertion axis of the FPC, and the FPC ripples or undergoes a pulling force even if the FPC is arranged as in the prior art examples. This poses a problem such that a disconnection is prone to occur, and the bending angle cannot be made large in all directions.

Additionally, in the prior art examples, it is important to keep the resistance force (expansion/contraction force) caused by the FPC in balance. As a result, not only must a physician apply a great amount of operation force, but also the bending tube undergoes a change such that it becomes difficult to carefully perform fine operations within a body cavity susceptible to damage. This results in the operational performance of the endoscope becoming worse, and the burden on the physician increases.

The bending tube of the endoscope can also be put into a complex bending state (twisting state) in which, for example, it is bent 90 degrees to the left and 130 degrees in the upward direction. This leads to a problem in which not only does the amount of bending operation force become greater but also the balance of the resistance force caused by the FPC is lost in the configuration of the prior art examples, and an operation different from that intended by the physician may occur.

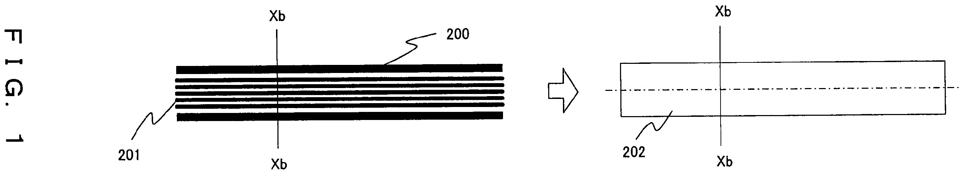

Alternately, if the FPC is not used--for example, if a signal line bundle 201 bound by a binding member (such as an outer sheath) 200 is used as shown in Fig. 1--the signal line bundle 201 is tightened by the binding member 200 so that the signal line bundle 201 becomes rigid part 202 . This leads to the problem that the amount of operation force that must be placed on the bending tube becomes great, and the operational performance becomes worse.

In particular, if the bending tube is bent with a curvature radius R, the center of the signal line bundle becomes a neutral axis, signal lines near the outer circumference of the bend undergo a pulling force and those near the inner circumference of the bend undergo a compression force due to the solidity of the signal line bundle. However, since the periphery of the bundle is bound, pressure is applied to the signal lines at the periphery. Accordingly, stress is repeatedly applied when an endoscopic operation is being performed, leading to a possible disconnection.

In consideration of the above described problems, the present disclosure provides an ultrasound endoscope that can have a large bending angle, a small amount of operation force, and superior functionality for insertion into a patient and in being operated by a physician.

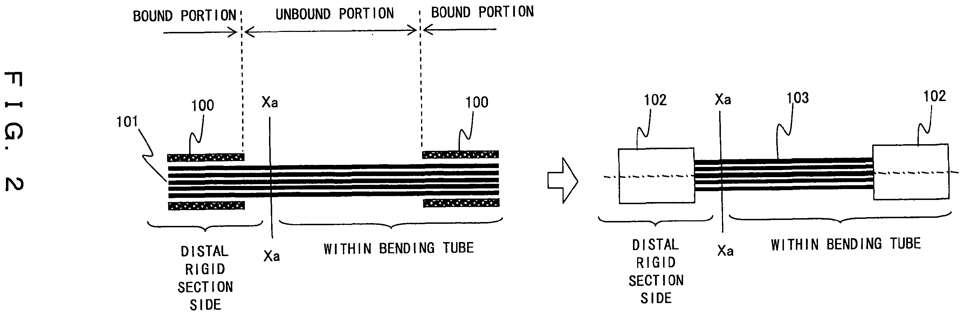

Fig. 2 is a conceptual schematic of bound portions and an unbound portion of a signal line bundle included in a bending tube of the ultrasound endoscope according to an example not forming part of the present invention. The signal line bundle 101 inserted into an insertion tube of the endoscope is covered with a binding member 100 over its total length, and is in a bound state (bound portion). The binding member 100 that covers the signal line bundle 101 is removed from a portion of the bending tube on the side containing the distal rigid section, and the signal line bundle 101 is bare. Accordingly, the signal line bundle of that portion is not bound by the binding member (unbound portion).

The right side of Fig. 2 shows a model of the rigid part of the left schematic of Fig. 2. In the right schematic of Fig. 2, the bound portions of the left schematic of Fig. 2 are represented as rigid parts 102, and the unbound portion is represented as a soft part 103.

Here, a comparison is made between a portion of an Xa-Xa line section of Fig. 2 and its corresponding Xb-Xb line section of Fig. 1. Line section Xb-Xb exhibits solidity, whereas line section Xa-Xa exhibits flexibility. Accordingly, the load imposed when the signal line bundle of Fig. 2 is bent is relatively lighter than that of Fig. 1.

The ultrasound endoscope according to the example not forming part of the present invention comprises an ultrasound probe part, which is arranged in the distal rigid section among the flexible tube, the bending tube, and the distal rigid section that configure the tip of the endoscopic insertion tube, and in which a plurality of ultrasound transducers transmitting ultrasounds vertically to the insertion axis are arranged in the form of a ring, and further comprises a bundle of signal lines that are inserted into the insertion tube, and the number of which is almost the same as the number of ultrasound transducers that transmit/receive signals to/from the ultrasound probe. The bundle of signal lines are situated inside the flexible tube, the bending tube, and the distal rigid section, which configure the tip of the endoscopic insertion tube.

Therefore, in this ultrasound endoscope, each of the signal lines extending from the ultrasonic probe is bound within the distal rigid section and the flexible tube , and at least a portion of the bending tube is bound with a weaker binding force than that of the binding within the distal rigid section and the flexible tube. As a result, each of the signal lines can move freely in the direction of the insertion axis of the endoscope, and can flexibly bend even if signal lines near the outer circumference undergo a pulling force and those near the inner circumference undergo a compression force as a result of the bending of the bending tube.

Additionally, signal lines up to one half of the total length of the entire bending tube are weakly bound within the binding tube. Namely, signal lines up to one half, which exists on the tip side, of the total length of the entire bending tube are weakly bound within the binding tube.

Preferred embodiments are described below.

### < 1-1 preferred embodiment>

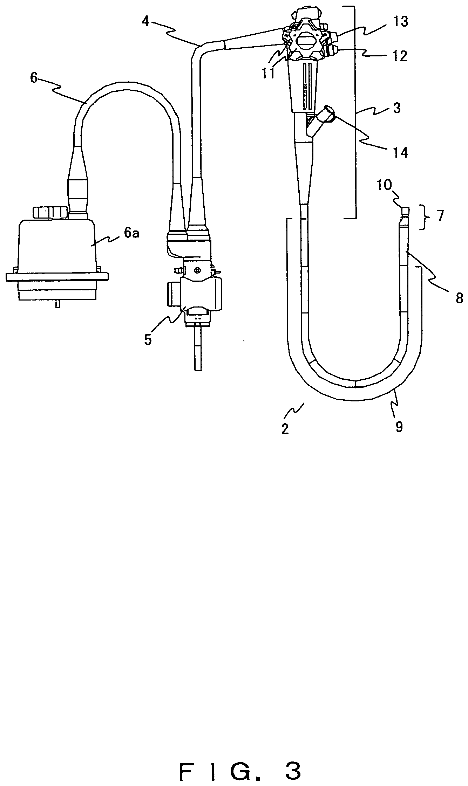

Fig. 3 shows the configuration of the outside of the ultrasound endoscope according to an example not forming part of the present invention. The ultrasound endoscope 1 is configured mainly with a long and thin insertion tube 2 for insertion into a body cavity, an control section 3 positioned at the base of the insertion tube 2, and a universal cord 4 extending from the side containing the control section 3.

Inside the universal cord 4, a light guide cable, a suction tube, an electric line, or other such items pass through. At the base of the universal cord 4, a scope connecter 5 connected to a light source device not shown is provided. From the scope connector 5 extends an electric cable that is freely connectable/ disconnectable to/from a camera control unit (not shown) via an electric connector. Additionally, from the scope connector 5 extends an ultrasonic cable 6 that is freely connectable/disconnectable to/from a Diagnostic ultrasound system (not shown) via an ultrasonic connector 6a.

The insertion tube 2 is configured by providing a rigid tip 7, a bending tube 8, and a flexible tube 9 in series. The distal rigid section 7 is formed with resin members that are harder nearer the tip and get less hard in sequence as they get further from the tip. The bending tube 8 is a tube that is positioned at the rear end of the distal rigid section 7, and can freely bend. The flexible tube 9 is a long and thin tube that is positioned at the rear end of the bending tube 8, and has flexibility. In the area nearer the tip of the distal rigid section 7, an ultrasonic probe 10 is provided. In the ultrasonic probe 10, a plurality of piezoelectric elements for transmitting/receiving ultrasounds are arranged.

In the control section 3, an angulation control knob 11, an air/water valve 12, a suction valve 13, and a instrument channel port 14, etc. are provided. The angulation control knob 11 is intended to control the bending of the bending tube 8 in a desired direction. The air/water valve 12 is intended to perform an air/water supply operation. The suction valve 13 is intended to perform a suction operation. The instrument channel port 14 is a port into which an endo-therapy accessorie to be guided into a body cavity enters.

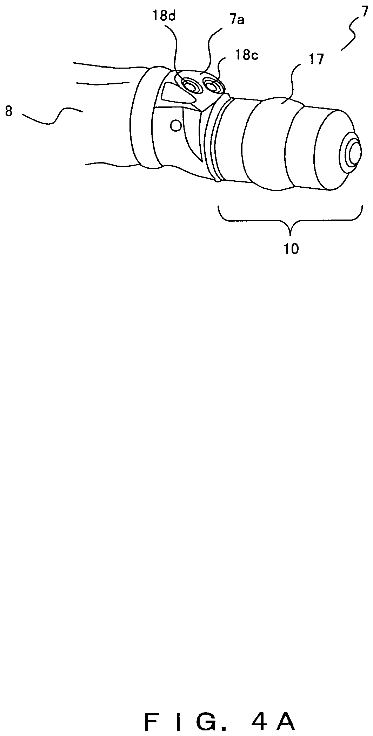

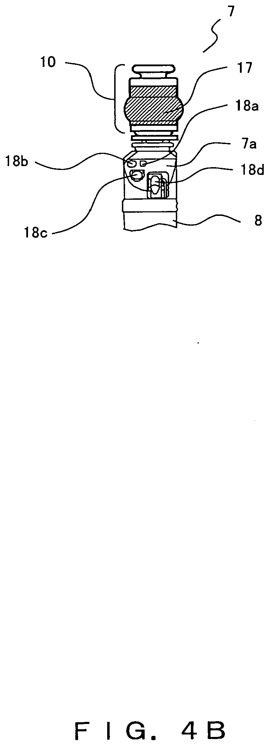

Figs. 4A and 4B are enlarged views of the distal rigid section 7 of the ultrasound endoscope 1 shown in Fig. 3. Fig. 4A is an external perspective view, whereas Fig. 4B shows its external configuration. At the tip of the distal rigid section 7, the ultrasonic transducer 10 that enables electronic radial scanning is provided. The ultrasonic transducer 10 is covered with a material with which acoustic lens (ultrasound transmitting/ receiving unit) 17 can be formed. Additionally, a slanting area 7a is formed in the distal rigid section 7. On the slanting area 7a, an illumination lens 18b, an objective lens 18c, a instrument-channel-outlet/suction-channel 18d, and an air/water supply port 18a are provided. The illumination lens 18b configures an illumination optical part for radiating illumination light on a portion to be observed. The objective lens 18c configures an observation optical part for capturing the optical image of the portion to be observed. The instrument-channel-outlet/suction-channel 18d is an opening from which an ablated portion is suctioned or an endo-therapy accessorie extends. The air/water supply port 18a is an opening for supplying air/water.

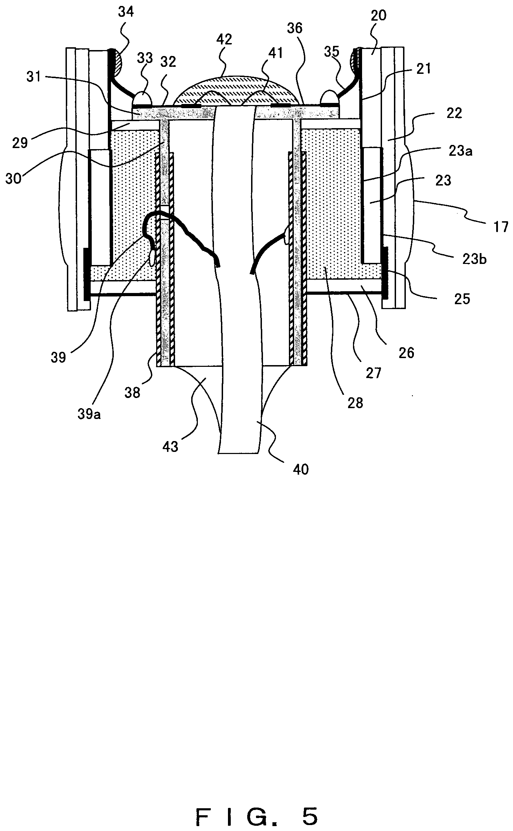

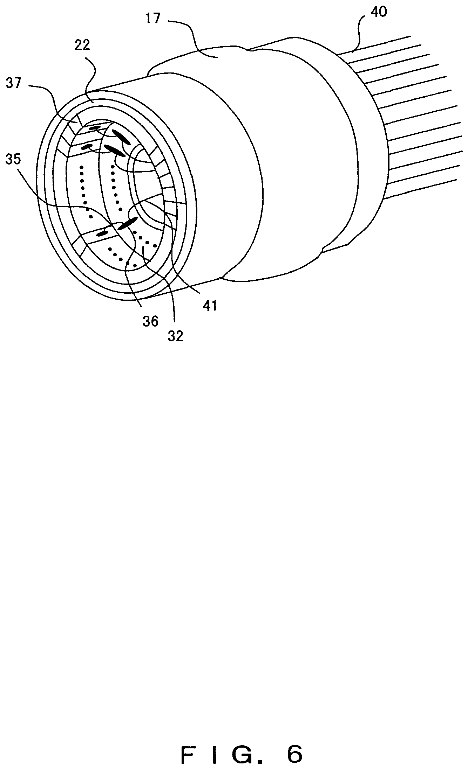

Fig. 5 shows a cross section of the ultrasonic probe, whereas Fig. 6 shows a perspective view. This preferred embodiment is described by taking an electronic radial ultrasonic probe as an example of the ultrasonic probe. The electronic radial ultrasonic probe is intended to transmit/receive an ultrasonic beam in a circumferential direction.

The ultrasonic probe 10 is shaped like a cylinder. The ultrasonic probe 10 is covered with an acoustic lens material 17 and an acoustic matching layer 22 in this order from the outermost circumference.

On opposing surfaces (surfaces at the inner and the outer portions of the ultrasonic probe 10) of a piezoelectric element 23, electrode layers 23a and 23b are respectively formed. Additionally, a conductive layer 21 is formed on one surface of a substrate 20 (the surface nearer the inner side of the ultrasonic probe 10). The conductive layer 21 and the electrode layer 23a are electrically connected. A conductive layer 25 continuous to the electrode layer 23b is formed in a portion of the acoustic matching layer.

As shown in Figs. 5 and 6, the substrate 20, conductive layer 21, piezoelectric element 23, electrode layer 23a, electrode 23b, conductive layer 25, and acoustic matching layer 22 (its portion) are diced, and a plurality of transducer elements 37 are formed.

Near openings on the bottom and the top sides of the ultrasonic probe 10, donut-shaped structural members 26 and 29 are respectively provided. The area between the structural members 26 and 29 is filled with a backing material 28. On the surface of the structural member 26 (the surface on the bottom side in this figure), copper foil is formed. The electrode 23b and the copper foil 27 are electrically connected via the conductive layer 25.

A cylindrical structural member 30 is inserted into the side at which the top opening is provided (the side on which the substrate 20 is provided). This cylindrical structural member 30 is configured with a cylindrical portion and a ring-shaped collar 31 provided on its one end. The collar 31 and the structural member 29 are joined, whereby the position of the cylindrical member 30 is fixed within the ultrasonic probe 10.

On the surface of the collar 31, a printed circuit board 32 is provided. A plurality of electrode pads 36 are provided on the surface of the printed circuit board 32. Additionally, a cable bundle 40 is inserted into the cylindrical structural member 30. The tip of each cable 41 of the cable bundle 40 is soldered to an electrode pad 36 corresponding to each cable 41. A coaxial cable is normally used as the cable 41 for noise reduction. Each of the electrode pads 36 is electrically connected to the conductive layer 21 via soldering and a wire 35. Potting is made with resin 42 for the cables 41.

The surface of the cylindrical portion of the cylindrical structural member 30 is covered with a metal thin film 38. A ground line 39 extending from the cable 40 is soldered with soldering 39a to the surface of the cylinder on which the metal thin film 38 is formed. Additionally, the metal thin film 38 is electrically connected to the copper foil 27.

As described above, the signal line of each cable 41 is electrically connected to one electrode 23a of a piezoelectric element of the transducer element 37, which corresponds to a signal line of each cable 41. Electrode 23b, as opposed to the signal electrode 23a of the piezoelectric element 23, is a ground electrode. The cable bundle 40 is bonded only near the opening of the cylindrical structural member 30 with an adhesive 43, and is bundled and bound.

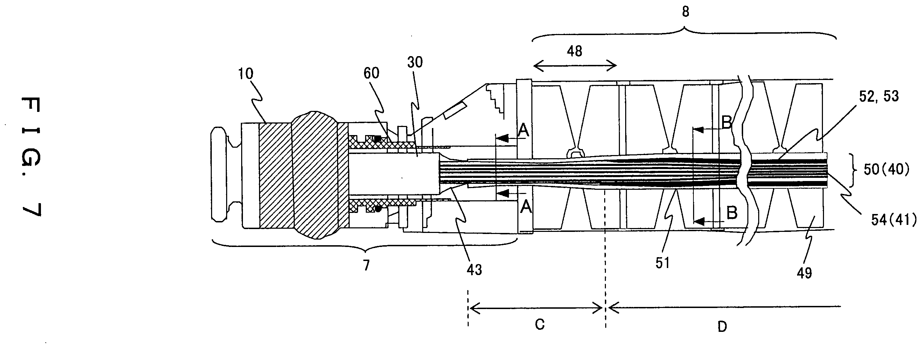

Fig. 7 shows a cross section near the tip of the insertion tube 2 of the ultrasound endoscope according to this preferred embodiment. The distal rigid section 7 and the bending tube 8 extend from the tip of the insertion tube 2. Additionally, a connection member 60 for joining the ultrasonic probe 10 to the tip structural member is provided.

Inside the connection member 60, the cylindrical portion of the cylindrical structural member 30 is housed. The cable bundle 40 extends from the opening of the cylindrical structural member 30, and passes through the inside of the bending tube 8.

Additionally, a series of linkage members (not shown) for bending the bending tube 8 in the left and the right directions (the direction vertical to the paper plane in Fig. 7), and a series of linkage members 49 for bending the bending tube 8 in the upward and the downward directions (the direction horizontal to the paper plane in Fig. 7) exist within the bending tube 8.

Furthermore, the bending tube 8 is configured with a plurality of bending modules 48. Accordingly, the linkage members operate, whereby the respective bending module 48 move, and the whole of the bending tube 8 bends. In this preferred embodiment, a multi-core coaxial cable 50 is used as the cable bundle 40.

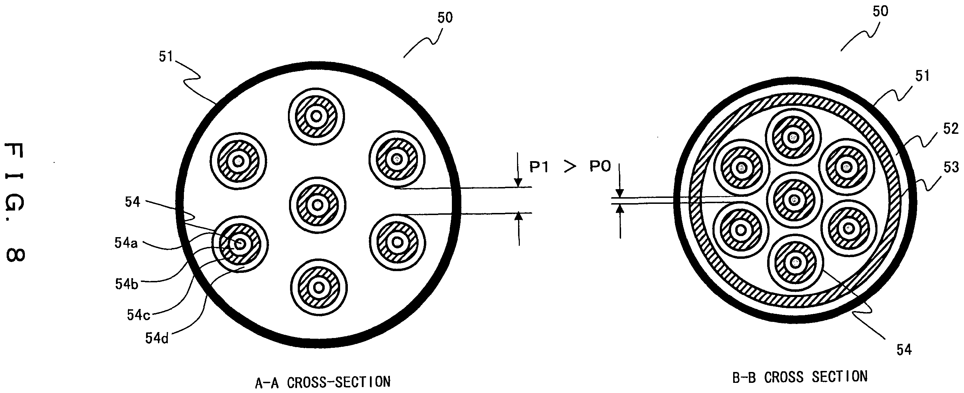

Fig. 8 shows the cross section of the multi-core coaxial cable 50 in this preferred embodiment. An A-A cross section of Fig. 8 is a schematic of the cross section of the multi-core coaxial cable 50, which corresponds to the cross section A-A of Fig. 7. A B-B cross section of Fig. 8 is a schematic of the cross section of the multi-core coaxial cable 50, which corresponds to the cross section B-B of Fig. 7.

The multi-core coaxial cable 50 is a bundle of a plurality of coaxial cables 54. In this preferred embodiment, the multi-core coaxial cable 50 has the cross section represented by the B-B cross section over its total length except for a portion to be described later (see the A-A cross section of Fig. 8). The B-B cross section of Fig. 8 is described first.

On the B-B cross section of Fig. 8, the core line (signal line) 54a of each coaxial cable 54 is configured by being covered with an insulator 54b, is also covered with a shield line 54c thereon, and is further covered with a jacket (outer sheath) 54d. Additionally, a plurality of coaxial cables 54 are bundled together, covered with a shield line (overall shield line) 53 thereon, and further covered with a jacket (outer sheath) 52. This is the configuration of a typical multi-core coaxial cable.

In this preferred embodiment, such a multi-core coaxial cable 50 is further covered with a heat-shrinkage tube 51. As described above, a predetermined number (a number corresponding to the number of transducer elements) of signal lines (signal line bundles) connected to the ultrasonic probe 10 are covered with the heat-shrinkage tube 51 over the jacket 52 over the insertion tube 2's total length within a portion of the distal rigid section 7, the bending tube 8, and he flexible tube 9.

The A-A cross section of Fig. 8 is implemented by removing the jacket 52 and the overall shield line 53 of the multi-core coaxial cable 50, which are described with reference to the B-B section of Fig. 8, and by covering the bundle of the bare coaxial cables 54 with the heat-shrinkage tube 51. Here, the reason that the bundle of coaxial cables is covered with the heat-shrinkage tube 51 is to prevent the coaxial cables 54 from being unbundled and to hold them with a certain degree of freedom. If any of the coaxial cables 54 were unbundled, it could possibly become stuck in the above described linkage members 49 or other such place and be disconnected.

Additionally, the heat-shrinkage tube 51 is also intended to align the multi-core coaxial cable 50 in a predetermined position within the bending tube 8. Furthermore, the heat-shrinkage tube 51 is also intended to improve safety by preventing the overall shield line 53 from being externally exposed when the jacket 52 is mechanically damaged on the B-B cross section of Fig. 8.

Referring back to Fig. 7, the jacket 52 and the overall shield line 53 are removed (portion C) from the portion of the bending tube 8 that is nearer the cylindrical structural member 30 in the multi-core coaxial cable 50 extending from the opening of the cylindrical structural member 30 toward the bending tube 8. The remaining portion is covered with the jacket 52 and the overall shield line 53 (portion D).

Then, the bundle of the bare coaxial cables 54 is bonded with the adhesive 43 in the vicinity of the opening of the cylindrical structural member 30, and is bundled and bound. The multi-core cable 50 is covered with the heat-shrinkage tube 51 over its total length, including also the entire portion (portion C) of the bundle of the bare coaxial cables 54 except for the bonded portion 43.

Incidentally, when the multi-core coaxial cable 50 is covered with the heat-shrinkage tube 51, the multi-core coaxial cable 50 is inserted into the heat-shrinkage tube 51 and is externally heated. A heat gradient is applied by varying heating time or by varying heating power or the heat source (for example, by using a small heater such as a heat gun or a large heating appliance, or by moving the heat source away from the object to be heated) so that the binding force of the heat-shrinkage tube 51 for each coaxial cable 54 is adjusted depending on the portion of the tube.

For example, the heating time for the heat-shrinkage tube 51 positioned in the portion (portion C) from which the jacket 52 and the overall shield line 53 are removed is made to be shorter than the other portions to relatively weaken the binding force for each coaxial cable 54.

By covering it with the heat-shrinkage tube 51, the signal lines (coaxial cables 54) within the jacket 52 (within the overall shield 53) are further bound (portion D). Accordingly, the interval P0 between coaxial cables 54 is narrowed as indicated by the B-B cross section of Fig. 8. As a result, the twisting of the overall shield 53 generates a high slide resistance in the signal lines (coaxial cables 54), and the respective signal lines (coaxial cables 54) are securely bound.

Since the jacket 52 and the overall shield 53 do not exist in portion C within the bending tube 8, binding for the signal lines (coaxial cables 54) is weak. Namely, as indicated by the A-A cross section of Fig. 8, the interval P1 between coaxial cables 54 becomes wide in comparison with the interval PO of the B-B cross section of Fig. 8 (P1>P0).

Accordingly, the degree of freedom of each coaxial cable 54 on the A-A cross section of Fig. 8 is higher than that in the case of the B-B cross section of Fig. 8. This indicates that the binding force for each coaxial cable 54 is smaller on the A-A cross section of Fig. 8 when the multi-core coaxial cable 50 is bent.

As described above, if the signal line bundle bends with a curvature radius R with the bending operation of the endoscope, signal lines positioned on the side of the inner circumference bow, and those positioned on the side of the outer circumference bend with the curvature radius R. Therefore, an unnecessary pulling force is not applied. Additionally, since an unnecessary force is not applied to the signal lines, not only is a disconnection prevented from occurring but also the signal line bundle becomes soft, leading to a reduction in the amount of operation force.

### <1-2 preferred embodiment>

If the signal line bundle is in a weakly bound state (a state in which the signal line bundle is not fully bound with the jacket 52, the overall shield line 53, etc., or a state of portion C in which the signal line bundle is covered only with the heat-shrinkage tube in the first preferred embodiment), the signal lines bow in various directions. As a result, the signal lines touch other included components (such as a light guide cable, etc.) existing within the bending tube, and an unnecessary load is imposed on the components. Accordingly, the length of the signal line bundle in the weakly bound state within the bending tube is reduced to one half of the total length of the bending tube in this preferred embodiment.

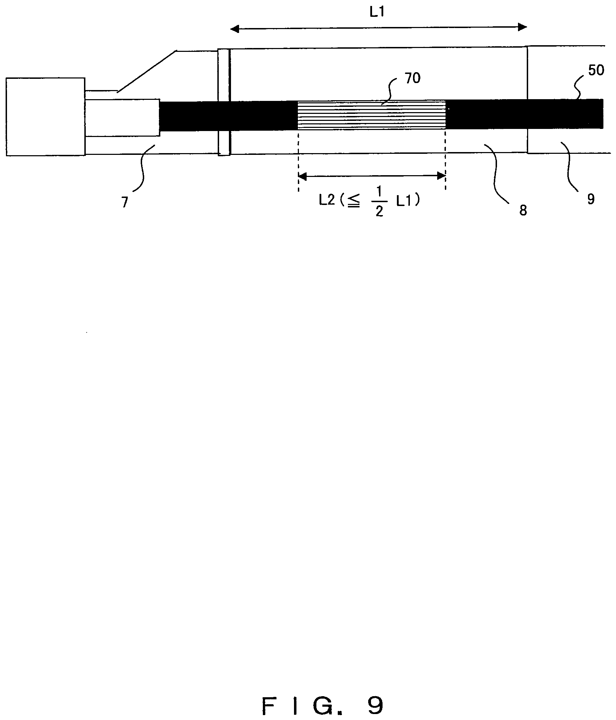

Fig. 9 is a schematic showing the tip of the insertion tube of the ultrasound endoscope according to this preferred embodiment. In this figure, assume that the total length of the bending tube and the length of the signal line bundle portion 70 in the weakly bound state are L1 and L2 respectively. Also, assume that L2 is equal to or smaller than one half of L1. For example, L2 may be set to a length of one third of L1.

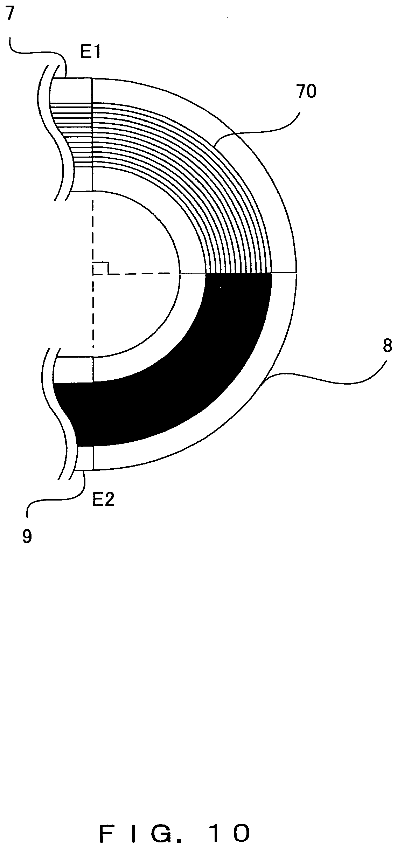

Fig. 10 shows the state of the signal line bundle in a case in which the bending tube 8 of the ultrasound endoscope according to this preferred embodiment is bent. If the bending tube 8 is bent in the form of a half circle so that one end E1 and the other end E2 of the bending tube 8 form a 180-degree angle, the limitation of the length of the signal line bundle portion 70 is represented by a 90-degree arc. If the arc is 90 degrees or more, the signal lines can possibly bow in various directions. Accordingly, it is better to set the signal line bundle portion in the weakly bound state to one half or less of the total length of the bending tube in consideration of Fig. 10.

According to this preferred embodiment, the region in which each signal line freely deforms can be restricted. Accordingly, interference with other included components such as a forceps channel or an optical observation member is prevented, and not only the disconnection of a signal line but also damage to other included components can be minimized.

### <1-3 preferred embodiment>

In the second preferred embodiment, the length of the signal line bundle portion in the weakly bound state within the bending tube is made to be equal to or smaller than one half of the length of the bending tube. In this preferred embodiment, a signal line bundle in the weakly bound state is made to be equal to or smaller than one half, which exists on the tip side, of the length of the side of the bending tube. It is sufficient that the tip of the insertion tube of the endoscope can bend flexibly and that the other portions only track the tip when the endoscope is guided into a body cavity.

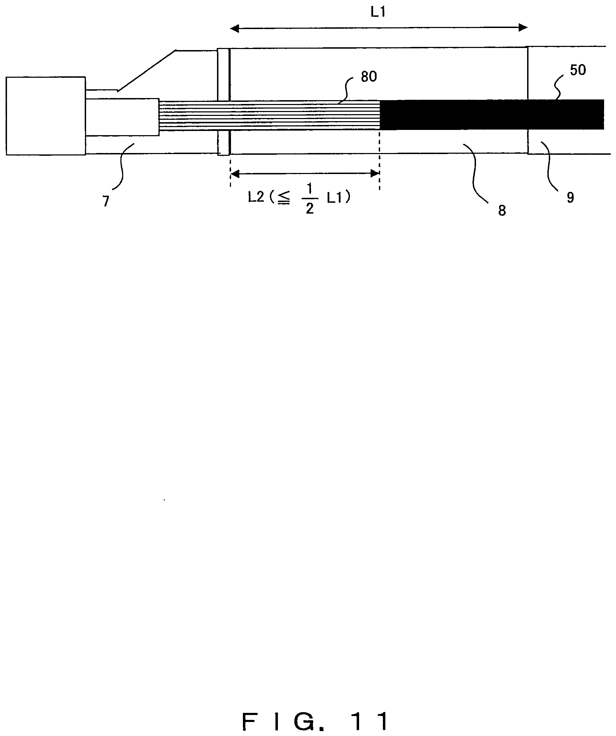

Fig. 11 is a schematic showing the tip of the insertion tube of the ultrasound endoscope according to this preferred embodiment. According to this figure, a flexible portion of the bending tube (a signal line bundle portion 80 in the weakly bound state) is positioned on the side nearer the tip.

As a result, a tracking capability into a lumen is enhanced when the endoscope is inserted into a patient, and its insertion performance is improved, thereby reducing the burden on the patient and physician.

In the 1-1 to 1-3 preferred embodiments, the heat-shrinkage tube is used. However, the binding member is not limited to the heat-shrinkage tube, and any member may be available as far as it can bind the signal line bundle. For example, a heat-shrinkage tape or other such device may be used. Furthermore, in the 1-1 to 1-3 preferred embodiments, the electronic radial ultrasonic probe is used. However, the ultrasonic probe is not limited to this type. For example, a convex or a linear ultrasonic probe may be used. Furthermore, in the 1-1 to 1-3 preferred embodiments, the electronic radial ultrasonic probe using a piezoelectric element is used. However, the ultrasonic probe is not limited to this type. An electronic radial ultrasonic probe using a capacitance transducer (c-MUT) is also applicable.

Still further, the present disclosure is not limited to the 1-1 to the 1-3 preferred embodiments. Diverse configurations can be adopted within the scope recited by the claims. Accordingly, inasmuch as binding force implemented by the cover member that covers the signal line bundle within a predetermined range in the signal line bundle included in the bending tube can be made smaller than that of the cover member which covers the other portions, this cover member is not limited to the configuration of the heat-shrinkage tube 51, the jacket 52, and the overall shield 53.

As described above, the bending angle can be made large and the amount of operation force can be reduced, whereby an ultrasound endoscope which imposes less burden on a patient and a physician can be obtained.

### <second preferred embodiment>

Since a conventional ultrasound endoscope does not comprise a cable fixing member for fixing an ultrasonic cable, it is possible for several tens or hundreds of wires to be twisted by bending stress or to be disconnected by tension at the time of assembly of the endoscope or at the time of an endoscopic diagnosis. Additionally, to provide the endoscope with the functions of an endoscopic observation part and an ultrasonic observation part within an optical system, difficulties exist in the thinning of the endoscope.

Additionally, the technique recited in Patent Document 4 implements a configuration in which at least a cable in a rigid part is made to extend toward an control section in parallel to an insertion axis, and this technique does not take into account an observation optical system.

An object of the present invention is to provide an electronic radial ultrasound endoscope the operational performance of which is improved and that reduces the burden on patients and physicians in consideration of the above conventional background.

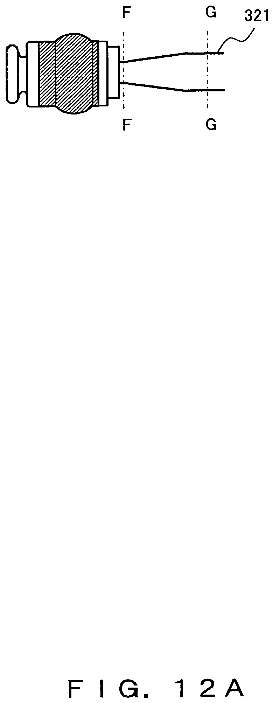

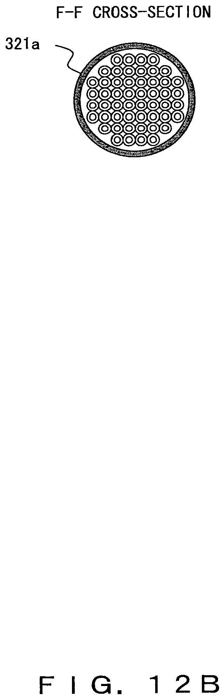

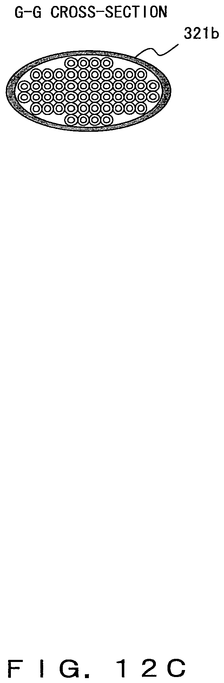

Figs. 12A, 12B, and 12C show the cable of the distal rigid section 7 of the electronic radial ultrasound endoscope 1 in this preferred embodiment (implementation example 1). The cable is configured by bounding ultrasonic transducer elements and signal lines for transmitting/receiving a driving signal. An F-F cross section of a cable 321 in the external view shown in Fig. 12A is shown in Fig. 12B. The cable 321a is shaped like a circle. Fig. 12C shows a G-G cross section of the cable 321 of Fig. 12A. The cable 321b is shaped by deforming a circle with a cable fixing member (not shown) shaped like an ellipse (a shape that is thin in the direction in which an ability to bend is desired) . The cable 321b may be made to easily bend in upward and downward directions in Fig. 12 by filling in at least a portion of the cable 321b with flexible resin.

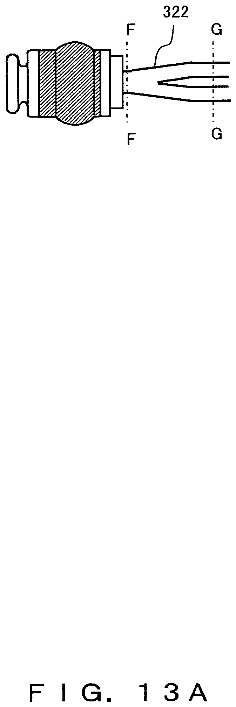

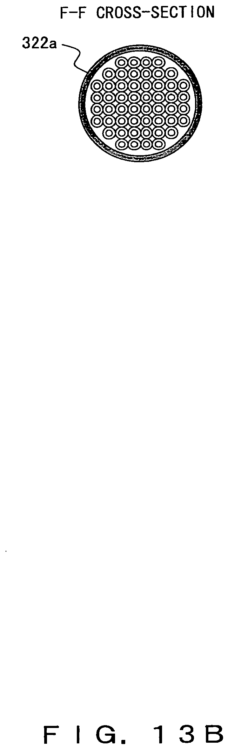

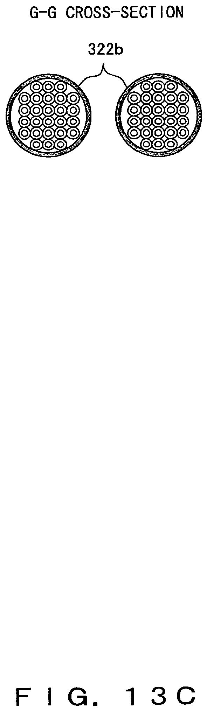

Figs. 13A, 13B, and 13C show the cable of the distal rigid section 7 of the electronic radial ultrasound endoscope 1 in this preferred embodiment (implementation example 2) . An F-F cross-sectional view of the cable 322, the external configuration of which is shown in Fig. 13A, is depicted in Fig. 13B. Cable 322a of the cable 322 is shaped like a circle. Fig. 13C shows the G-G cross section of the cable 322 shown in Fig. 13A. Cable 322b is branched into two branches that are easy to bend in a desired bending direction (the upward and downward directions shown in Fig. 13C) . Cable 322b may be branched not only into two branches but also into three branches. There are no problems if cable 322b is thin in the direction in which the ability to curve is desired and is thus easy to curve.

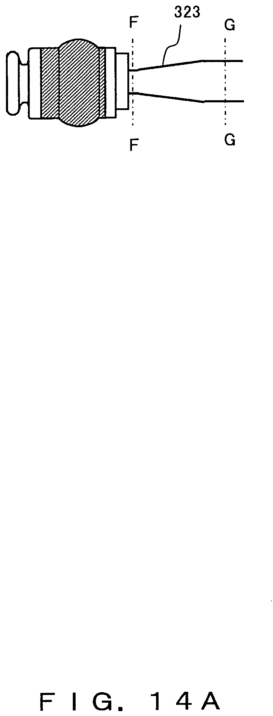

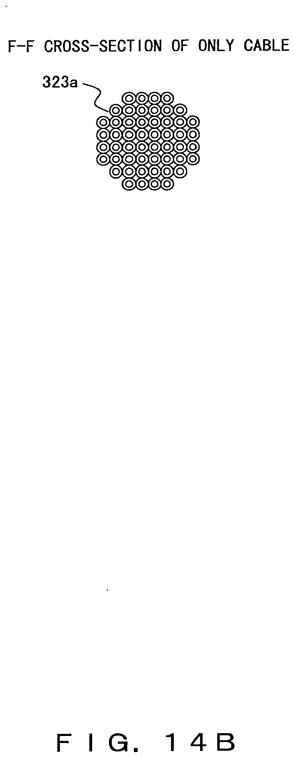

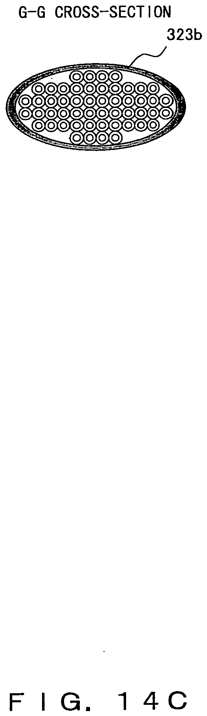

Figs. 14A, 14B, and 14C are schematics showing the cable of the distal rigid section 7 of the electronic radial ultrasound endoscope 1 in this preferred embodiment (implementation example 3). An F-F cross section of the cable 323, the external configuration of which is shown in Fig. 14A, is depicted in Fig. 14B. The cable 323a is shaped like a circle for which the outer circumference is not covered in the area near a transducer. Fig. 14C shows the G-G cross section of the cable 323 shown in Fig. 14A. In a similar manner as in Fig. 12C, the cable 323 is shaped like, for example, an ellipse (thin in a predetermined direction in which the ability to bend is desired) due to a cable fixing member not shown by being deformed from a circle to an ellipse. If necessary, the cable may be made easy to bend in the upward and the downward directions of Fig. 14C by filling at least a portion of the cable 323b with flexible resin.

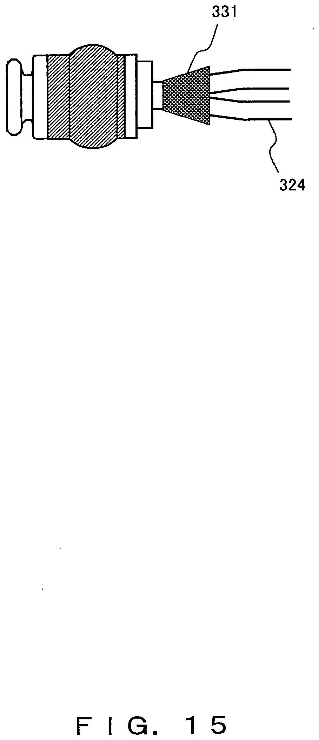

Fig. 15 is a schematic showing a state where a heat-shrinkage tube is attached to the cable in this preferred embodiment. The heat-shrinkage tube 331 for preventing the two-branched cable 324 from being disconnected is made of, in this example, fluorine resin. To branch the cable 324, it is desirable to attach the heat-shrinkage tube 331 to a branched point.

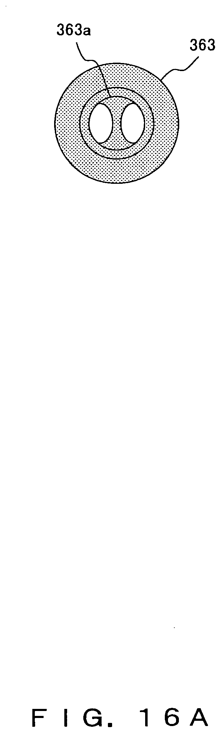

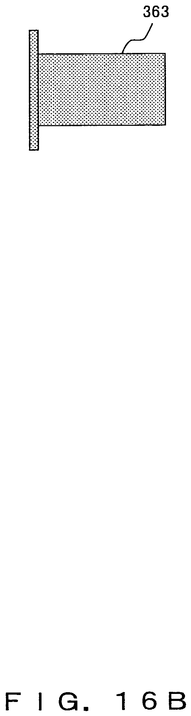

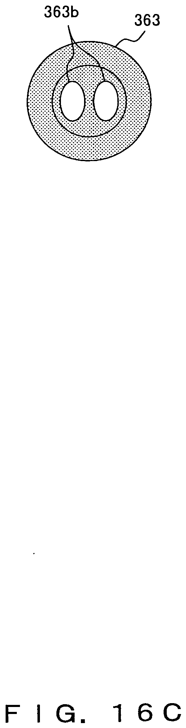

Figs. 16A, 16B, and 16C exemplify a cable fixing member 363 for fixing the cable 324 shown in Fig. 15. Fig. 16A is a schematic when Fig. 15 is viewed in the left direction. In Fig. 16A, one cable hole 363a is formed on the side containing the tip of the cable. Fig. 16B is a schematic when Fig. 15 is viewed in the same direction as that of Fig. 15. Fig. 16C is a schematic when Fig. 15 is viewed in the right direction. As shown in Fig. 16C, two cable holes 363b are formed to fix the branched cables.

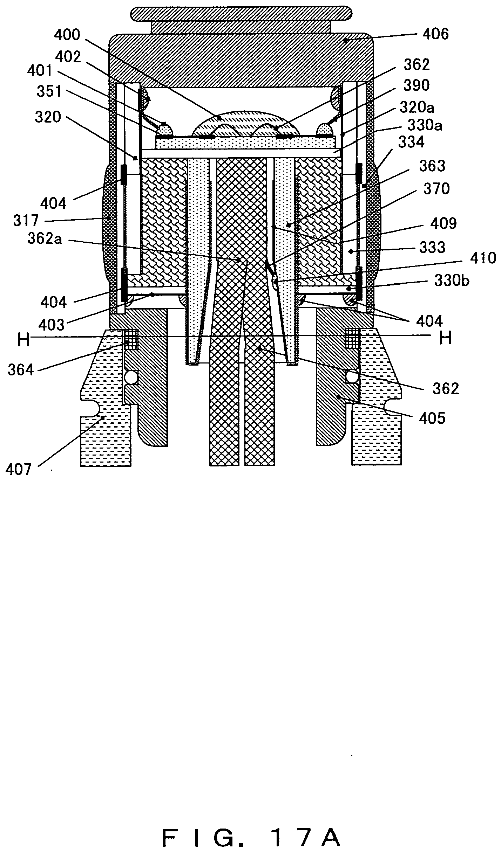

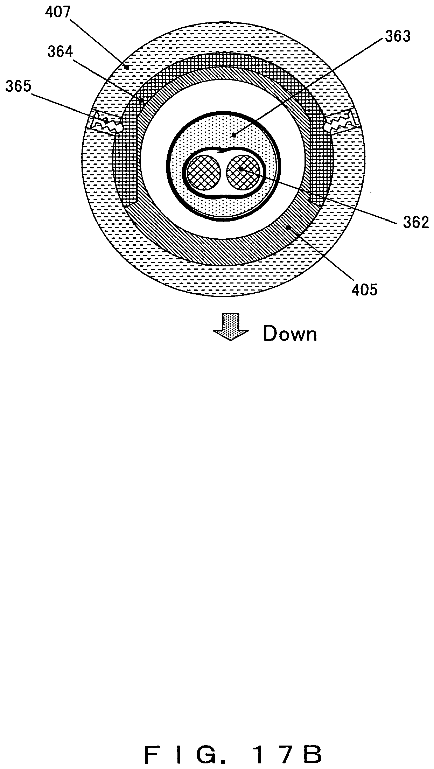

The state of the cables fixed by the cable fixing member 363 within the distal rigid section 7 is described next with reference to Figs. 17A and 17B.

Fig. 17A is a cross-sectional side view of the distal rigid section 7 of the electronic radial ultrasound endoscope 1 shown in Fig. 13. A signal line 362 is connected to the side of the central direction of a collar in the electrode pad 351. One end of a wire 390 is connected with soldering 401 to the outer circumference of the collar in the electrode pad 351, whereas the other end is connected with soldering 402 to a signal side electrode 320a positioned on the substrate 320 of the transducer element. A short wire 390 is used and connected in order to prevent the wire from short circuiting by touching the adjacent signal side electrode 320a. Additionally, the whole of the connected portion of the signal line 362 and the electrode pad 351 is covered with potting resin 400 in order to prevent the signal line 362 from coming off from the electrode pad 351 by being pulled with a load imposed on the signal line 362. The signal line 362 branches into two branches from a cable branched portion 362a, and a cable fixing member 363 is provided on the outer circumference of the signal line 362. Additionally, copper foil 403 is formed on one surface of the structural member 330b, and a conductive film 409 is formed on the side of the cylinder of the cable fixing member 363. A ground line 370 that collects the ground lines of the cable 362 composed of a plurality of signal lines 362 is connected with soldering 410 to the conductive film 409 provided on the side of the cylinder of the cable fixing member 363, and is further linked to the electrode on the surface of a transducer of a piezoelectric element 333 via the copper foil 403 on the surface of the structural member 330b and a conductive resin layer 404 formed by providing grooves in an acoustic matching layer 334. On the side of an acoustic transmission surface of the piezoelectric element, the acoustic matching layer 334 and an acoustic lens 317 are arranged.

After this ultrasonic transducer is manufactured, a tip structural member 406 and a structural member 405 are connected to it, and the ultrasonic transducer is fixed to a distal rigid section 407 with a screw 365 by using a U-shaped alignment member 364, so that the distal rigid section 7 of the ultra sound endoscope 1 is formed.

Fig. 17B is an H-H cross-sectional view of the distal rigid section 7 shown in Fig. 17A. The electronic radial ultrasonic transducer 10 is configured by forming grooves with the dicing of a piezoelectric element on the acoustic matching layer 334 in the form of a flat plate, and is made cylindrical. Therefore, a joint between one end and the other end of the flat plate is aligned in the down direction shown in Fig. 17B. This is because image precision can be degraded due to pitch, misalignment, and slight differences in components of the joint. The alignment member 364 is provided at a desired angle on the outer circumference of the structural member 405, and the distal rigid section 407, which is fixed with a fixing screw 365 to the alignment member 364, is provided.

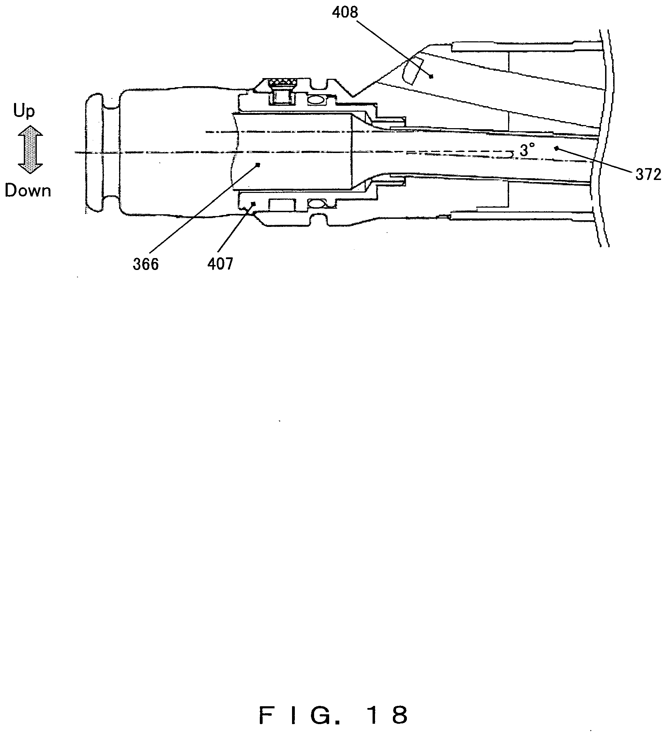

Fig. 18 is a cross-sectional side view showing the inclination of the cable of the distal rigid section 7 in this preferred embodiment. As shown in this figure, the cable 372 is inclined (here, for example, by 3 degrees) in a direction away from an observation optical system 408, which is an endoscopic observation part.

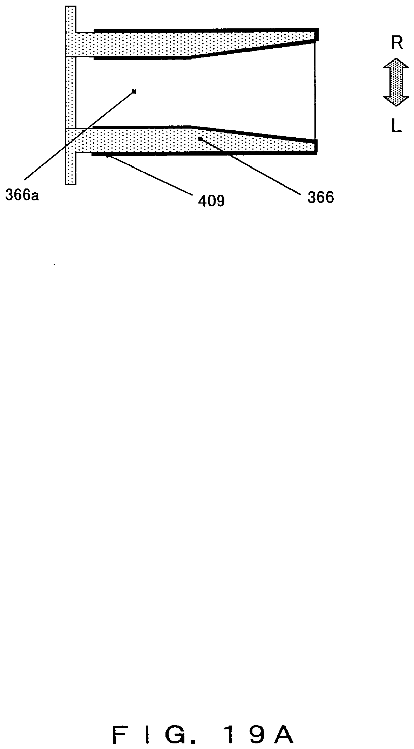

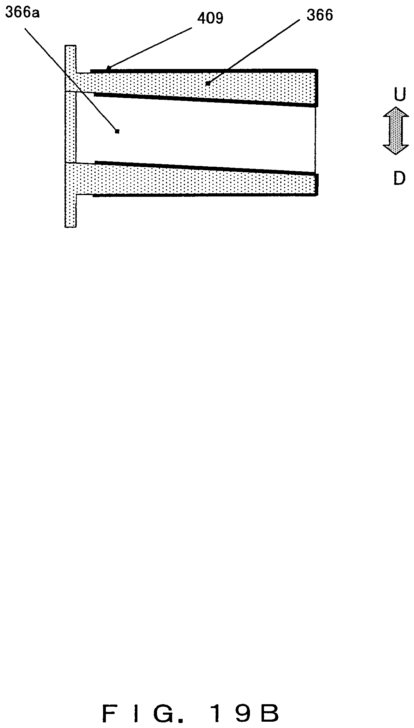

Figs. 19A and 19B show a cable fixing member 366 for inclining the cable 362 in this preferred embodiment. Fig. 19A shows the shape of the endoscopic observation part in the R (right) and L (left) orientations. Fig. 19B shows the shape in the U (upward: the are in which the observation optical system exists) and D (downward) orientations. A cable hole 366a of the cable fixing member 366 having a surface to which a conductive film 409 is joined is inclined in the D direction from the side of the tip of the cable (the left side in the figure).

Operations in this preferred embodiment are described next.

Like the cable 321b shown in Fig. 12C, the cable is shaped to be thin in a desired bending direction (upward and downward directions in the figure) by being transformed from a circle to an ellipse with a cable fixing member not shown, whereby bending in a desired direction becomes easy.

The thickness of the cable in a desired bending direction is made to be thin when the cable is branched like the cable 322b shown in Fig. 13C, whereby bending in a desired bending direction becomes easy. Additionally, the easy bending direction is made to match the UP and the DOWN directions of the optical observation part and the ultrasonic observation part, whereby the cable can be bent in a direction intended by a physician when he or she rotates the angulation control knob 11 of the endoscopic control section.

Like the cable 323a shown in Fig. 14B, an uncovered cable may also be used in the vicinity of the distal rigid section. In this case, if the surface of the cable fixing member is metal-plated, the cable is grounded by connecting the overall shield, which extends from the uncovered portion, to the cable fixing member. As a result, electric noise is not exerted, and the cable is electrically safe to a human body.

As shown in Fig. 15, the cable 324, which is branched into two bundles covered with the heat-shrinkage tube 331, is combined with the cable fixing member 363 shown in Fig. 16, whereby tension applied to the signal line 362 shown in Fig. 17 can be removed. This is because the tension of the cable, which is applied to the side of nearer the transducer, is absorbed by the heat-shrinkage tube 331 in the branched portion of the cable fixing member. For this reason, stress applied to each signal line 362 is significantly reduced, no wire to the substrate is ever disconnected, and a highly reliable ultrasound endoscope can be manufactured. In this preferred embodiment, the cable fixing member is provided with the function for changing the shape of the cable. However, other similar effects may be produced such that the cable stress that occurs at the time of bending is not applied to the side of nearer the transducer within not only the distal rigid section 407 but also the distal rigid section 7, and a direction in which bending is easy can be identified by the shape of the cable.

Additionally, as shown in Fig. 19B, the cable hole 366a of the cable fixing member 366 is inclined from the side of the tip toward the D (down) direction, whereby the available area of the observation optical part becomes wide and the degree of freedom in arrangement increases.

As described above, according to this preferred embodiment, a cable composed of a bundle of signal lines connected to respective ultrasonic transducers is fixed to be thin in a preset direction in the distal rigid section or the bending tube, whereby bending in a predetermined direction becomes easy and the burden on patients and physicians is reduced.

Furthermore, the central axis of the cable is inclined in a direction away from the observation optical system relative to the orientation of its insertion axis, whereby the area available to the observation optical system is broadened and the degree of freedom of arrangement increases. As a result, further thinning can be produced and the burden on patients and physicians is reduced. The thinning also improves the operational performance. In this preferred embodiment, the central axis of the cable is inclined in the cable fixing member. Similar operations and effects can also be obtained with a structure that uses a hole provided with a slope for inclining the cable or that uses a circular guide in the distal rigid section without providing an inclination to the cable fixing member.

Still further, a joint when the electronic radial ultrasonic transducer is formed to be cylindrical is fixed in a position opposed to the observation optical system, whereby the joint at which image precision is degraded is arranged in a position that is not used often by a physician and is opposed to the observation optical system, and diagnostic accuracy is improved.

Still further, the heat-shrinkage tube is comprised in a cable branched position when the branched cable is fixed, whereby a disconnection, which is caused by stress applied to the cable, can be prevented.

Still further, this preferred embodiment is not limited to the ultrasonic transducer using a piezoelectric element, and is also applicable to an electronic radial ultrasonic transducer using a capacitance transducer (c-MUT).

As described above, according to the electronic radial ultrasound endoscope according to the present invention, a cable composed of a bundle of signal lines connected to respective ultrasonic transducer elements is fixed to be thin in a preset orientation in the distal rigid section, whereby bending in a predetermined direction becomes easy and the burden on patients and physicians is reduced.

Additionally, the central axis of the cable is inclined such that it gets further away from the observation optical system as it gets closer to the insertion axis of the cable, whereby the area available to the observation optical part is widened and the degree of freedom of arrangement increases. As a result, further thinning can be achieved and the burden on patients and physicians is reduced. The thinning also improves operational performance.

Furthermore, the joint when the electronic radial ultrasonic transducer is formed to be cylindrical is fixed in a position opposed to the observation optical system, whereby image precision is degraded in positions that are not used often by physicians and is opposed to the observation optical part, and diagnostic accuracy is improved.

Still further, the heat-shrinkage tube is comprised in the branched portion of the cable when the branched cable is fixed, whereby a disconnection caused by tension applied to the cable can be prevented.

## Claims

1. An electronic radial ultrasound endoscope (1) comprising
an insertion tube (2) which is configured by providing a distal rigid section (7), a bending tube (8), and a flexible tube (9) in series, wherein
the distal rigid section (7) configures a tip part of the insertion tube (2), the distal rigid section (7) including
an ultrasonic transducer (10) shaped as a cylinder at the tip of the distal rigid section (7) which has a plurality of ultrasonic transducer elements (37) for transmitting/receiving ultrasounds, and
an endoscopic observation part (18c) where an illumination optical system (18b) and an observation optical system (408) are provided, wherein
the bending tube (8) is configured to bend freely at least in mutually orthogonal first and second bending directions and is positioned at the proximal end of the distal rigid section (7), a cable (321, 322, 323, 324, 362, 372) composed of a bundle of signal lines connected to the respective ultrasonic transducer elements (37) **characterised in that** the thickness of said cable (321,322,323,324,362,372) is thinner in the first bending direction than in the second bending direction in the distal rigid section (7),
a joint between one end and the other end of a flat plate made cylindrical forming an acoustic matching layer (334) covering the ultrasonic transducer (10) is fixed in a position opposed to the observation optical system (408), and
the distal rigid section (7) is formed by a connection of a tip structural member (406) and a structural member (405) to the ultrasonic transducer (10), and by a fixation of the ultrasonic transducer (10) to a part (407) of the distal rigid section (7) with a screw (365) by means of an U-shaped alignment member (364).

2. The electronic radial ultrasound endoscope (1) according to claim 1, wherein the cable (324, 362, 372) is fixed with a cable fixing member (363, 366) provided within the ultrasonic transducer (10).

3. The electronic radial ultrasound endoscope (1) according to claim 2, wherein a shape of the cable (324, 362) is deformed with the cable fixing member (363).

4. The electronic radial ultrasound endoscope (1) according to claim 2, wherein a central axis of the cable (372) is inclined with the cable fixing member (366) in a direction away from the observation optical system (408) relative to an insertion axis direction of the cable (372).

5. The electronic radial ultrasound endoscope (1) according to claim 2, wherein the cable fixing member (363, 366) is a structural member for connecting the endoscopic observation part (18c) and the ultrasonic transducer (10).

6. The electronic radial ultrasound endoscope (1) according to one of claims 1 and 4 to 5, wherein the cable (322, 324, 362) is branched, and a thickness of the branched cables (322b, 324, 362) is thinner in the first bending direction than in the second bending direction in the distal rigid section (7).

7. The electronic radial ultrasound endoscope (1) according to claim 6, wherein the branched cable (322b, 324, 362) is fixed with a cable fixing member (363) provided within the ultrasonic transducer (10).

8. The electronic radial ultrasound endoscope (1) according to claim 6 or 7, wherein a heat-shrinkage tube (321, 331) is attached to a branched portion of the cable (324, 362).

## Patentansprüche

1. Elektronisches Radial-Ultraschallendoskop (1) umfassend
ein Einführrohr (2), das durch Anordnen eines distalen starre Abschnitts (7), eines Biegerohrs (8) und eines flexiblen Rohrs (9) in Reihe gebildet wird, wobei
der distale starre Abschnitt (7) einen Spitzenteil des Einführrohrs (2) ausbildet, wobei der distale starre Abschnitt (7) umfasst
einen als ein Zylinder geformten Ultraschall-Wandler (10) an der Spitze des distalen starren Abschnitts (7), der eine Vielzahl von Ultraschall-Wandlerelementen (37) zum Senden/Empfangen von Ultraschall aufweist, und
einen endoskopischen Beobachtungsteil (18c), in dem ein optisches Beleuchtungssystem (18b) und ein optisches Beobachtungssystem (408) angeordnet sind, wobei
das Biegerohr (8) ausgebildet ist, sich frei wenigstens in gegenseitig orthogonaler erster und zweiter Biegerichtung zu biegen und am proximalen Ende des distalen starren Abschnitts (7) angeordnet ist,
ein aus einem Bündel von mit den entsprechenden Ultraschall-Wandlerelementen (37) verbundenen Signalleitungen bestehendes Kabel (321, 322, 323, 324, 362, 372), **dadurch gekennzeichnet, dass** die Dicke des Kabels (321,322,323,324,362,372) in der ersten Biegerichtung dünner ist als in der zweiten Biegerichtung im distalen starren Abschnitt (7),
eine Verbindung zwischen einem Ende und dem anderen einer zylindrisch gestalteten flachen Platte, die eine akustisch passende Schicht (334) zum Abdecken des Ultraschall-Wandlers (10) bildet, in einer Position gegenüber dem optischen Beobachtungssystem (408) befestigt ist, und
der distale starre Abschnitt (7) durch eine Verbindung eines Spitzenstrukturelements (406) und eines Strukturelements (405) mit dem Ultraschall-Wandler (10) und durch eine Befestigung des Ultraschall-Wandlers (10) an einem Teil (407) des distalen starren Abschnitts mit einer Schraube (365) mit Hilfe eines u-förmigen Ausrichtungselements (364) gebildet wird.

2. Elektronisches Radial-Ultraschallendoskop (1) nach Anspruch 1, wobei das Kabel (324, 362, 372) mit einem im Ultraschall-Wandler (10) angeordneten Kabelbefestigungselement (363, 366) befestigt ist.

3. Elektronisches Radial-Ultraschallendoskop (1) nach Anspruch 2, wobei eine Form des Kabels (324, 362) mit dem Kabelbefestigungselement (363) verformt wird.

4. Elektronisches Radial-Ultraschallendoskop (1) nach Anspruch 2, wobei eine Mittelachse des Kabels (372) mit dem Kabelbefestigungselement (366) in einer Richtung weg vom optischen Beobachtungsystem (408) relativ zu einer Einführachsenrichtung des Kabels (372) schräggestellt ist.

5. Elektronisches Radial-Ultraschallendoskop (1) nach Anspruch 2, wobei das Kabelbefestigungselement (363, 366) ein Strukturelement zum Verbinden des endoskopischen Beobachtungsteils (18c) und des Ultraschall-Wandlers (10) ist.

6. Elektronisches Radial-Ultraschallendoskop (1) nach einem der Ansprüche 1 und 4 bis 5, wobei das Kabel (322, 324, 362) verzweigt ist und eine Dicke der verzweigten Kabel (322b, 324, 362) dünner in der ersten Biegerichtung ist als in der zweiten Biegerichtung im distalen starren Abschnitt (7).

7. Elektronisches Radial-Ultraschallendoskop (1) nach Anspruch 6, wobei das verzweigte Kabel (322b, 324, 362) mit einem im Ultraschall-Wandler (10) angeordneten Kabelbefestigungselement (363) befestigt ist.

8. Elektronisches Radial-Ultraschallendoskop (1) nach Anspruch 6 oder 7, wobei ein Wärmeschrumpfschlauch (321, 331) an einem verzweigten Teil des Kabels (324, 362) befestigt ist.

## Revendications

1. Endoscope à ultrasons radial électronique (1) comprenant :
un tube d'insertion (2) qui est configuré par disposition d'une section rigide distale (7), d'un tube de flexion (8), et d'un tube flexible (9) en série, dans lequel :
la section rigide distale (7) configure une partie d'embout du tube d'insertion (2), la section rigide distale (7) comprenant :
un transducteur ultrasonore (10) en forme de cylindre à l'embout de la section rigide distale (7) qui présente une pluralité d'éléments transducteurs ultrasonores (37) pour émettre/recevoir des ultrasons, et
une partie d'observation endoscopique (18c) où un système optique d'éclairage (18b) et un système optique d'observation (408) sont disposés, dans lequel :
le tube de flexion (8) est configuré pour se fléchir librement au moins dans de première et seconde directions de flexion mutuellement orthogonales et est positionné à l'extrémité proximale de la section rigide distale (7),
un câble (321, 322, 323, 324, 362, 372) composé d'un faisceau de lignes de signal connectées aux éléments transducteurs ultrasonores (37) respectifs,
**caractérisé par le fait que** l'épaisseur dudit câble (321, 322, 323, 324, 362, 372) est plus mince dans la première direction de flexion que dans la seconde direction de flexion dans la section rigide distale (7),
une articulation entre une extrémité et l'autre extrémité d'une plaque plate rendue cylindrique formant une couche d'adaptation acoustique (334) recouvrant le transducteur ultrasonore (10) est fixée en une position opposée au système optique d'observation (408), et
la section rigide distale (7) est formée par une liaison d'un élément structural d'embout (406) et d'un élément structural (405) au transducteur ultrasonore (10), et par une fixation du transducteur ultrasonore (10) à une partie (407) de la section rigide distale (7) avec une vis (365) au moyen d'un élément d'alignement en forme de U (364).

2. Endoscope à ultrasons radial électronique (1) selon la revendication 1, dans lequel le câble (324, 362, 372) est fixé avec un élément de fixation de câble (363, 366) disposé à l'intérieur du transducteur ultrasonore (10).

3. Endoscope à ultrasons radial électronique (1) selon la revendication 2, dans lequel une forme du câble (324, 362) est déformée avec l'élément de fixation de câble (363).

4. Endoscope à ultrasons radial électronique (1) selon la revendication 2, dans lequel un axe central du câble (372) est incliné avec l'élément de fixation de câble (366) dans une direction à l'opposé du système optique d'observation (408) par rapport à une direction d'axe d'insertion du câble (372).

5. Endoscope à ultrasons radial électronique (1) selon la revendication 2, dans lequel l'élément de fixation de câble (363, 366) est un élément structural pour une liaison de la partie d'observation endoscopique (18c) et du transducteur ultrasonore (10).

6. Endoscope à ultrasons radial électronique (1) selon l'une des revendications 1 et 4 à 5, dans lequel le câble (322, 324, 362) est ramifié, et une épaisseur des câbles ramifiés (322b, 324, 362) est plus mince dans la première direction de flexion que dans la seconde direction de flexion dans la section rigide distale (7).

7. Endoscope à ultrasons radial électronique (1) selon la revendication 6, dans lequel le câble ramifié (322b, 324, 362) est fixé avec un élément de fixation de câble (363) disposé à l'intérieur du transducteur ultrasonore (10).

8. Endoscope à ultrasons radial électronique (1) selon la revendication 6 ou 7, dans lequel un tube thermorétractable (321, 331) est fixé à une partie ramifiée du câble (324, 362).
